# EUROPEAN PATENT SPECIFICATION

(11) **EP 0 977 530 B1**
(45) Date of publication and mention of the grant of the patent: **16.07.2003**
(21) Application number: 98920773.3
(22) Date of filing: 22.04.1998
(51) Int. Cl.: A61F 5/10, A61F 5/37, A61F 13/10

(54) **SPLINT**
SCHIENE
ATTELLE

(30) Priority: 25.04.1997 SE 9701570
(43) Date of publication of application: 09.02.2000
(73) Proprietor: Rehband Anatomiska AB, 191 07 Sollentuna (SE)
(72) Inventor: FERNHOLM, Christine, S-183 34 Täby (SE); ERIKSSON, Thomas, S-135 50 Tyresö (SE)
(74) Representative: Janson, Ronny
(86) International application number: SE9800733
(87) International publication number: WO98048744

(56) References cited:
- FR-A- 2 578 416
- US-A- 4 949 711
- US-A- 5 376 066

## Description

This invention concerns an ulnar deviation splint for correction of ulnar deviated hands.

Ulnar deviation result in the wrist and the fingers of a patient being turned sideways in the direction of the little finger side. As seen from the upper side of the hand and the wrist there is, thus, at least in grave cases, a double sideward turn, which in an advanced stage gravely impairs the function of the hand and particularly its gripping capacity. Because of the sideways turn of the MCP-joints of the hand, that is the inner joints between the fingers and the rest of the hand, a oblique draw of the wrongly directed tendons occur, resulting in wear in this region.

The subluxation of the MCP joints, meaning that the joint surfaces slide away from each other, is also serious, and typically the fingers "fall down" relative to the remaining part of the hand. This occurs because inflammation in the joint result in expansion of the joint-capsule and instability in the joint.

Two kinds of splints for correction of ulnar deviation are previously known, on the one hand a chute-shaped splint which follows the shape of the lower arm and the hand and which is intended to be used during resting and thereby gives the wrist, the hand and the fingers a corrected straightened position.

For use in an active situation, a snorted version of the above splint has been used, a so called work splint, which, however, is uncomfortable and ineffective.

Its an aim of this invention to provide a splint for relieving and correcting ulnar deviation with the aid of a splint which is experienced as comfortable and effective by the patient and which provides at least partial re-established function of the hand in question.

This aim is obtained through an ulnar deviation splint according to claim 1.

Thanks to the splint according to the invention it is achieved on the one hand the possibility to correct already existing deformation, for example ulnar displacement of fingers or subluxation of the MCP joints. The splint also provides a clearly enhanced function of the hand and fingers of the user by providing them with a corrected functional position at the same time as strength as well as moveability are enhanced. This results in that the patient in a normal manner will be able to grip and hold items of different kinds. Further, the splint prevents continued deterioration of the incorrect position being caused by ulnar deviation since the hand will be held in a corrected position and thereby joints will be relieved.

Further, the splint according to the invention functions as a preventive measure by holding joints in a functional position so as to prevent or at least limit the creation of deformations, for example ulnar displacement of fingers and subluxation of MCP joints.

In the case where ulnar deviation is less severe, MCP correction with the aid of the upwardly adjustably tension means will be sufficient, whereas in graver cases it is also necessary to have a three point contact by means of a longitudinal stiffening means - an "ulnar rail" ( compare claim 2).

In the most severe case a "volar rail" is used, that is a support means which is inserted at the lower side of the splint which further in a advantageous way provides correcting and supporting effect to the hand.

The adjustable tension means which are arranged to pass most inwardly in the space between two adjacent fingers, make possible, by their adjustability and there tensioning pulling effect upwardly and rearwardly, a lifting effect on the fingers with respect to the rest of the hand which brings about correction of the above mentioned descent of the fingers. In greater detail the fingers are thereby lifted from a position where they tend to be a continuation of the plane through the palm of the hand to a more normal position where the fingers are a continuation of the rest of the hand. Subluxation of the MCP joints are, thus, prevented, that is, the tendency of the joint surfaces to slide apart is prevented. At the same time, already by the draw or pull upwardly, rearwardly between the knuckles, a certain straightening of the ulnar deviated MCP joints is obtained, resulting in enhanced function of the hand and reducing wear by oblique draw. In order for the tension means to be able to fulfil this function, they do not have too great transverse dimensions, since this would be troublesome for the finger function. They are therefore comprised of "strings" or round bands having a thickness of usually about 3 - 6 mm.

By each adjustable tension means also being adjustable so as to provide tension sideways, in the direction from the side of the little finger (claim 4), a further possibility of correcting sideways turned MCP joints is provided for somewhat more suffering patient.

Further advantages are achieved by the features in the other claims and will be clear from the following detailed description of an embodiment referring to the annexed drawing, wherein:
Fig. 1 shows a splint according to the invention seen from above,
Fig. 2 shows the splint of Fig. 1 seen from below, and
Fig. 3 shows the splint of Fig. 1 and 2 as seen in a perspective view from the finger side.

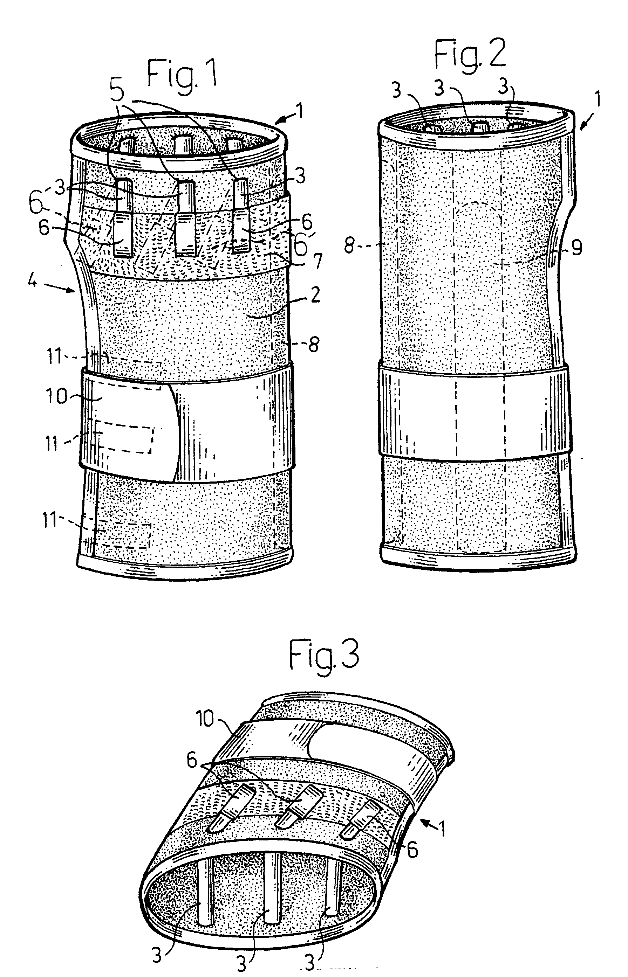

The splint 1 shown in Fig. 1 consists of a tubular flexible main body 2, which is adapted to be put onto the hand of a user in the same manner as a glove. When it is applied it is adapted to extend from a first point on the lower arm of the user, at a distance above the wrist to a second point, below, or outside, the MCP joints of the fingers. The dimension is of course depending on the size of the patient, but normal longitudinal extension is between 15 and 20 cm. The Figure shows the splint with the finger side pointing upwards on the paper.

The splint 1 of Fig. 1 is a right hand splint wherein the thumb is intended to penetrate generally in the region of arrow 4. At the upper part of the splint 1 there are arranged three adjustable tension means 3 which in use are adapted to be located most inwardly in the spaces between the fingers of the patient and which are firmly fixed to the inside of the main body, whereas they are drawn through holes, at 5, in the upper side of the main body. This construction makes tensioning of the tension means upwardly, rearwardly (full lines in Fig. 1) possible, and obliquely from the little finger side towards the thumb side, the latter being indicated with interrupted lines in Fig. 1. Fastening of the tension means are achieved by a fastening portion 6 which is applied at the end of each tension means, and is provided with Velcro fasteners, and is arranged to co-operate with an area 7 on the outside of the splint being provided with a textile layer for fastening said Velcro fastener. In use, the tension means are thus pulled rearwardly between the knuckles in the direction of the upper side of the wrist, whereby the above mentioned lifting and correcting function is obtained.

By the free possibility of sideward adjustment 6', the possibility is provided to correct sideways turned MCP joints according to the need of the individual patient.

At 8, interrupted lines indicate a stiffening means in the form of a "ulnar rail" which assure sideward alignment of the wrist. It is preferred that the length of the rail 8 is such that it extends between the MCP joint of the little finger and a point above the wrist of the user. This way a three point support of the wrist is obtained whereby the third point is the point where the splint contacts the wrist of the opposite side with respect to the location of the ulnar rail. The effect of this three point contact is enhanced by using a tensioning band 10 around the main body at this location. This rail is preferably made from a synthetic material which allows adaptation to the patient in question. A suitable material is Turbocast (registered trade mark), which is a so called thermo-formable material allowing simple shaping to the patient subsequent to heating with for example hot water.

Fig. 2 shows the lower side of the splint, wherein at 9, a support means or a volar rail is indicated which is inserted into a pocket in the splint and which provides support for the wrist against a downward turn of the hand. The support means is preferably simple to insert and to remove so that it may be inserted when needed, for example when resting, whereas it is removed in a work situation.

From Fig. 3 the configuration of the finger side of the splint with the three tension means 3 are shown more clearly.

The material of the different parts of the splint is not critical, but it is preferred that the main body is comprised of micro-fleece having a laminated'membrane of a suitable polymer. The ulnar rail is preferably of a thermo-plastic material whereas the volar rail is made of metal, preferably one which is simply formable for adjustment to different desired hand positions. It is also possible to insert rails of different lengths for different needs. In order to simplify putting on the splint, it is preferably slotted from region 4 and down (Fig. 1) and is provided with Velcro fasteners for simple locking which is indicated with number 11.

The invention thus provides an ulnar deviation splint for use in many different situations, such as at rest, as a night bandage and in different kinds of work, for example with monotonous and repetitive elements, and in moments of strain, where the fingers are held extremely at the direction of the little finger. Continued deterioration of inadequate positions are prevented by the hand being held in a correct position and joints being relieved.

## Claims

1. Ulnar deviation splint including:
- an generally tubular, flexible main body (2) which in use is arranged to extend from a first point on the lower arm of the user at a distance above the wrist, to a second point below the MCP joints of the fingers as seen with the arm hanging and with the fingers of the hand directed downwards, and
- three upwardly, rearwardly adjustable tension means (3) for passage essentially across the main plane of the hand closest to the MCP joints, between two adjacent fingers for exerting a lifting effect on the fingers with respect to the rest of the hand.

2. Splint according to claim 1, wherein an ulnar stiffening means (8) is arranged to be connected to the main body (2) and to have its respective end extending past the wrist of the user and which is adapted to be applied on the side of the little finger of the wrist, as seen from the upper side of the hand, for sideward support of the wrist.

3. Splint according to claim 2, wherein the stiffening means (8) is arranged so as to lie against the MCP joint of the little finger at one of its ends.

4. Splint according to any of the claims 1 - 3, wherein each adjustable tension means (3) is also adjustable so as to provide tension sideways in a direction from the side of the little finger.

5. Splint according to any of the previous claims, wherein a longitudinal support means (9) is fastenable on the lower side of the main body for support on both sides of the wrist between the palm and a point above the wrist.

6. Splint according to any of the claims 2 - 5, wherein the stiffening means (8) and/or on occurrence the support means (9) is inserted into a corresponding pocket in the main body.

7. Splint according to any of the previous claims, wherein each tension means (3) is fastenable on the main body (2) by Velcro fasteners (6, 7).

8. Splint according to any of the previous claims, wherein an adjustable tension band (10) is arranged around the periphery of the main body (2) in the region of the wrist.

## Patentansprüche

1. Schiene für Ulnarabweichung einschließend:
- einen im wesentlichen röhrenförmigen, flexiblen Hauptkörper (2), der im Gebrauch angeordnet ist, um sich von einem oberhalb des Handgelenks beabstandeten ersten Punkt am Unterarm des Benutzers zu einem zweiten Punkt unterhalb der MCP Gelenke der Finger erstreckt, bei herabhängendem Arm und nach unten gerichteten Fingern betrachtet, und
- drei aufwärts, rückwärts anpassbare Spannungseinrichtungen (3) für einen Durchgang im Wesentlichen durch die Hauptebene der Hand, die den MCP Gelenken am nächsten ist, zwischen zwei benachbarten Fingern, um eine Hebewirkung auf die Finger hinsichtlich der übrigen Hand zu bewirken.

2. Schiene nach Anspruch 1, wobei eine Ulnarversteifungseinrichtung (8) angeordnet ist, mit dem Hauptkörper (2) verbunden zu werden, und die Anordnung derart ist, dass sich deren jeweiliges Ende über das Handgelenk des Anwenders hinaus erstreckt, und die angepasst ist, auf der Seite des kleinen Fingers des Handgelenks, von der Oberseite der Hand aus betrachtet, für eine seitliches Unterstützen des Handgelenks angebracht zu werden.

3. Schiene nach Anspruch 2, wobei die Versteifungseinrichtung (8) derart angeordnet ist, um gegen das MCP Gelenk des kleinen Fingers an einem Ende davon anzustoßen.

4. Schiene nach einem der vorstehenden Ansprüche, wobei jede anpassbare Spannungseinrichtung (3) außerdem derart anpassbar ist, dass eine seitliche Spannung in eine Richtung von der Seite des kleinen Fingers bereitgestellt ist.

5. Schiene nach einem der vorstehenden Ansprüche, wobei eine längsseitige Stützeinrichtung (9) auf der unteren Seite des Hauptkörpers befestigt werden kann zum Unterstützen auf beiden Seiten des Handgelenks zwischen der Handfläche und einem Punkt oberhalb des Handgelenks.

6. Schiene nach einem der Ansprüche 2 bis 5, wobei die Versteifungseinrichtung (8) und/oder bei Vorhandensein die Stützeinrichtung (9) in eine entsprechende Tasche in dem Hauptkörper eingebracht wird.

7. Schiene nach einem der vorstehenden Ansprüche, wobei jede Spannungseinrichtung (3) mittels Klettverschlussmitteln (6,7) an dem Hauptkörper (2) befestigt werden kann.

8. Schiene nach einem der vorstehenden Ansprüche, wobei ein anpassbares Spannungsband (10) um den Umfang des Hauptkörpers (2) in dem Bereich des Handgelenks angeordnet ist.

## Revendications

1. Attelle pour déviation cubitale comprenant :
- un corps principal flexible, globalement tubulaire (2) qui, lors de l'utilisation, est agencé de façon à s'étendre depuis un premier point sur l'avant-bras de l'utilisateur sur une distance au-dessus du poignet, jusqu'à un second point en dessous des articulations MCP des doigts comme vu avec le bras pendant et avec les doigts de la main dirigés vers le bas, et
- trois moyens de traction (5) réglables vers l'arrière, orientés vers le haut, destinés à passer essentiellement à travers le plan principal de la main au plus près des articulations MCP, entre deux doigts adjacents, pour exercer un effet d'élévation sur les doigts par rapport à la partie restante de la main.

2. Attelle selon la revendication 1, dans laquelle un moyen de raidissement cubital (8) est agencé de façon à être relié au corps principal (2) et avoir son extrémité respective s'étendant au-delà du poignet de l'utilisateur, et qui est conçu pour être appliqué sur le côté du petit doigt du poignet, comme vu depuis le côté supérieur de la main, pour supporter de côté le poignet.

3. Attelle selon la revendication 2, dans laquelle le moyen de raidissement (8) est agencé de façon à s'étendre contre l'articulation MCP du petit doigt à l'une de ses extrémités.

4. Attelle selon l'une quelconque des revendications 1 à 3, dans laquelle chaque moyen de traction réglable (3) est également réglable de façon à appliquer une traction de côté dans une direction partant du côté du petit doigt.

5. Attelle selon l'une quelconque des revendications précédentes, dans laquelle un moyen de support longitudinal (9) peut être fixé au côté inférieur du corps principal pour constituer un support sur les deux côtés du poignet entre la paume et un point au-dessus du poignet.

6. Attelle selon l'une quelconque des revendications 2 à 5, dans laquelle le moyen de raidissement (8) et/ou en l'occurrence, le moyen de support (9) est inséré dans un logement correspondant dans le corps principal.

7. Attelle selon l'une quelconque des revendications précédentes, dans laquelle chaque moyen de traction (3) peut être fixé au corps principal (2) à l'aide d'organes de fixation (6, 7) du type Velcro.

8. Attelle selon l'une quelconque des revendications précédentes, dans laquelle une bande de traction réglable (10) est agencée autour de la périphérie du corps principal (2) dans la région du poignet.
